# EUROPÄISCHE PATENTSCHRIFT

(11) **EP 0 509 008 B1**
(45) Veröffentlichungstag und Bekanntmachung des Hinweises auf die Patenterteilung: **30.11.1994**
(21) Anmeldenummer: 91901647.7
(22) Anmeldetag: 03.01.1991
(51) Int. Cl.: C12N 5/06, A61K 39/12

(54) **BIOMASSE ZUR PRODUKTION VON VIRUS/VIRUSANTIGEN**
BIOMASS FOR THE PRODUCTION OF VIRUSES/VIRUS ANTIGENS
BIOMASSE POUR LA PRODUCTION DE VIRUS/ANTIGENES VIRAUX

(30) Priorität: 04.01.1990 AT 18/90
(43) Veröffentlichungstag der Anmeldung: 21.10.1992
(73) Patentinhaber: IMMUNO Aktiengesellschaft, A-1221 Wien (AT)
(72) Erfinder: MUNDT, Wolfgang, A-1080 Wien (AT); WÖHRER, Wilfried, A-2540 Bad Vöslau (AT); DORNER, Friedrich, A-1230 Wien (AT); EIBL, Johann, A-1180 Wien (AT)
(74) Vertreter: Wolfram, Gustav, Dipl.-Ing.
(86) Internationale Anmeldenummer: AT9100003
(87) Internationale Veröffentlichungsnummer: WO9109937

(56) Entgegenhaltungen:
- FR-A- 2 444 466
- US-A- 4 059 485
- US-A- 4 195 130
- ACTA VIROLOGICA, vol. 27, März 1983, Prag (CR); I. SLAVIK et al., Seiten 97-104#

## Beschreibung

Die Erfindung betrifft eine Biomasse und ein verfahren zur Produktion von Virus/Virusantigen.

Verfahren zur Herstellung von Virus/Virus-Antigen sind bekannt. Ausgangsmaterialien sind häufig sogenannte Primärzellkulturen, die aus menschlichen oder tierischen Geweben gewonnen werden. Diese Primärzellen werden mit Virus ("Saatvirus") infiziert und durch die Virusvermehrung wird Virusantigen gebildet.

Eine Methode zur Vermehrung beispielsweise des FrühsommerMeningoenzephalitis-Virus (FSME-Virus) wird in der AT -B 358.167 beschrieben: Hühnerembryonalzellen werden in einem Zellkulturmedium suspendiert, mit dem Virus infiziert und als Biomasse zur Produktion von FSME-Virusantigen verwendet. Dazu wird die Biomasse unter aeroben Bedingungen bei einer Temperatur zwischen 25 und 38 °C zwischen einem und fünf Tagen in Suspension gehalten. Dann werden die Zellen und Zellbruchstücke durch Zentrifugieren abgetrennt, die erhaltene Virussuspension mittels Formalin oder β-Propiolacton inaktiviert und das Virusantigen durch Ultrafiltration konzentriert, gereinigt und in üblicher Weise zu Vakzinen weiterverarbeitet.

Bei üblichen Präparationen von Primärzellkulturen wird besonders darauf Wert gelegt, Einzelzellen oder möglichst kleine Zellverbände zu erhalten. Um dies zu erreichen, muß das Gewebe möglichst stark mechanisch und enzymatisch zerkleinert werden. Die Behandlung führt jedoch gleichzeitig auch zum Absterben vieler Zellen. Werden solche Zellpräparationen an der Oberfläche geeigneter Trägermaterialien angesiedelt, bleiben tote Zellen im Überstand und können entfernt werden. Bei Verwendung solcher Zellpräparationen in Suspensionskulturen für die Herstellung von FSME-Virusantigen gibt es jedoch keine Möglichkeit, lebende Zellen von toten bzw. geschädigten Zellen zu trennen. Die mit der Zeit stattfindende Lyse der Zellen führt in der Folge zu einer hohen Verunreinigung an Zellproteinen im Medium, die schwer von dem gewünschten Produkt abgetrennt werden können.

Auch die Reproduzierbarkeit der Virus/Virusantigen-Herstellung ist bei Verwendung von Einzelzellen oder kleinen Zellaggregaten in Suspension gering, da es zu einer starken Zellschädigung z.B. durch Scherkräfte beim Rühren kommt.

Die Erfindung stellt sich die Aufgabe, diese Nachteile zu beseitigen und somit eine Biomasse zur Produktion von Virus/Virusantigen zur Verfügung zu stellen, welche bei Kultivierung eine hohe Produktionsleistung an Virus/Virusantigen entwikkelt, einfach zu handhaben ist und im großtechnischen Maßstab zur Produktion von Virus/Virusantigen verwendet werden kann, wobei das Virus/Virusantigen aus dem Kulturmedium in hoher Reinheit gewonnen werden kann.

Die erfindungsgemäße Biomasse, die den genannten Anforderungen entspricht, besteht aus Zellaggregaten mit einem Durchmesser zwischen 100µm und 1000µm, ist mit Virus infiziert.

Die Zellaggregate der erfindungsgemäßen Biomasse können durch mechanische und enzymatische Behandlung menschlicher oder tierischer Gewebe erhalten werden, wobei das auf mechanische Weise desintegrierte Gewebe zur weiteren Auflösung der Zellverbände mit einer Protease wie Trypsin, Chymotrypsin oder Elastase bis zur gewünschten Größe der Zellaggregate weiter zerkleinert werden kann.

Die Zellaggregate der erfindungsgemäßen Biomasse können aber auch aus menschlichen oder tierischen Einzelzellen erhalten werden, indem diese mit zellaggregierenden Substanzen, wie Agglutinin, behandelt werden.

Die Abtrennung von Zellaggregaten und Einzelzellen mit einem Durchmesser von größer als 1000µm bzw. kleiner als 100 µm kann durch Sieben oder vorzugsweise durch Sedimentieren vorgenommen werden. Die Sedimentation ist im Vergleich zur Siebung einfacher durchzuführen, da Siebe mit einer Porengröße von 100µm sehr leicht verstopfen. Weiters kommt die Sedimentation ohne aufwendige und teure Separatoren aus und bietet auch Vorteile in steriltechnischer Hinsicht. Es hat sich gezeigt, daß die Zellaggregate mit einem Durchmesser zwischen 100µm und 1000µm mit einer Geschwindigkeit von größer als 1 cm/min sedimentieren, während die kleineren Zellaggregate mit einer Geschwindigkeit von weniger als 1 cm/min sedimentieren. Die Abtrennung der Partikel mit einer Größe von kleiner als 1000 µm kann einfach mit Sieben erfolgen, da hier die Gefahr einer Verstopfung gering ist.

Eine bevorzugte Ausführungsform der erfindungsgemäßen Biomasse ist dadurch gekennzeichnet, daß sie in im Kulturmedium suspendiertem Zustand eine hohe metabolische Aktivität besitzen; gemessen am Glucoseverbrauch beträgt die metabolische Aktivität pro Stunde zwischen 3 und 5 mg Glucose pro Gramm Biomasse.

Die zur Bereitung der erfindungsgemäßen Biomasse verwendeten Zellaggregate besitzen weiters den Vorteil, daß sie bereits bei einer Infektion mit einer relativ kleinen Menge an Saatvirus große Mengen an Virusantigen produzieren. Es hat sich gezeigt, daß zur Infektion von Zellaggregaten, die kleiner als 100µm oder größer als 1000µm sind, wesentlich mehr Saatvirus verwendet werden muß, um die gleiche Menge an Virus/Virusantigen zu produzieren.

Die Virus/Virusantigenproduktion kann weiter gesteigert werden, wenn die erfindungsgemäße Biomasse bei einer Sauerstoffkonzentration von mindestens 0,01 mmol/l, vorzugsweise von mindestens 0,06 mmol/l im Kulturmedium gehalten wird.

Es ist vorteilhaft, wenn das Kulturmedium eine Konzentration an Zellaggregaten von mindestens 10mg Zellaggregate pro ml aufweist.

Die erfindungsgemäße Biomasse eignet sich besonders gut zur Produktion von Frühsommer-Meningoenzephalitis-Virus(FSME-Virus)/Virusantigen, wenn sie aus Zellaggregaten von Vogelembryonalzellen, insbesondere von Hühnerembryonalzellen besteht, wobei eine Virus/Virusantigen-Produktionsleistung weiter erhöht wird, wenn im Kulturmedium eine Sauerstoff-Transfer-Rate von größer als 1,60 mmol O₂.l⁻¹.h⁻¹, vorzugsweise zwischen 1,65 und 2,40 mmol O₂.l⁻¹.h⁻¹, eingestellt wird.

Die Sauerstoff-Transfer-Rate (oxygen transfer rate, OTR), ausgedrückt in mmol O₂.l⁻¹.h⁻¹, ist bekanntlich ein Maß für die Einbringung von Sauerstoff in die Zellkultur. Die Sauerstoff-Aufnahme-Rate (oxygen uptake rate, OUR) ist wiederum ein Maß für die metabolische Leistung einer Zelle allgemein und somit indirekt für die Virus/Virusantigensyntheseleistung einer Zelle.

Bei allen heute bekannten Methoden zur Produktion von FSME-Virusantigen ist die spezifische Produktionsleistung an Virus-Antigen beschränkt, da es bei Vorhandensein einer großen Menge an infizierten Zellen im Kulturmedium zu einem Absinken seines pH-Wertes und damit zu einer unerwünschten Inaktivierung des Virustiters und Zerstörung des Virusantigens kommen kann.

Es wurde gefunden, daß der pH-Wert einer solchen stark belüfteten Kultur nicht absinkt, und daß eine konstante und hohe Produktionsleistung an Virus/Virusantigen aufrecht erhalten werden kann, wenn in die Kulturflüssigkeit ausreichende Mengen an Sauerstoff z.B. durch Einrühren oder mittels statischer und/oder dynamischer Gasverteiler eingetragen werden.

Messungen haben ergeben, daß sich bei einer Kultur mit einer Zelldichte von 2 x 10⁷ Zellen pro Milliliter, die ausschließlich über ihre Oberfläche mit der Sauerstoff-Atmosphäre in Verbindung steht, die FSME-Produktion in größerem Maßstab stark reduziert. Wird Sauerstoff hingegen aktiv in die Kultur eingetragen, so kann die Antigenausbeute stark gesteigert werden.

Eine bevorzugte Ausführungsform des erfindungsgemäßen Verfahrens besteht darin, daß eine Sauerstoff-Transfer-Rate von größer als 1,65 mmol O₂.l⁻¹.h⁻¹, vorzugsweise zwischen 1,65 und 2,40 mmol O₂.l⁻¹.h⁻¹, eingestellt wird, wobei die Feuchtzellmasse am besten maximal 30 g pro Liter Kulturmedium beträgt.

Versuche haben ergeben, daß die FSME-Virus/Virusantigenproduktion der Konzentration an erfindungsgemäß verwendeten Zellaggregaten proportional ist. Das erfindungsgemäße Verfahren ist auch bei einer Zellaggregatkonzentration von weniger als 10mg/ml ausführbar. Im Gegensatz dazu wurde festgestellt, daß im Falle der Kultivierung von infizierten Einzelzellen eine Mindestkonzentration von 10mg/ml für eine Virus/Antigenproduktion unbedingt erforderlich ist.

Die zur Infektion einer Primärzellkultur benötigten hohen Saatvirustiter werden üblicherweise nur durch Vermehrung des Virus in Maushirn erreicht. Dadurch, daß Zellaggregate gemäß der Erfindung mit einer wesentlich geringeren Virusmenge infiziert werden können, kann das zur Infektion der Biomasse benötigte Saatvirus auch aus Zellkulturüberständen gewonnen werden. Dies verringert deutlich die Möglichkeit einer Kontamination mit Maushirnprotein.

Es hat sich weiters gezeigt, daß sich die erfindungsgemäße Biomasse bestehend aus Zellaggregaten von Vogelembryonalzellen ebenso zur Produktion von Influenza-, Vaccinia- oder Avipox-Virusantigen eignet.

Mit den nachfolgenden Ausführungsbeispielen wird die Erfindung noch näher erläutert.

### Beispiel 1: Einfluß der Zellaggregatgröße auf die Antigenausbeute

SPF-(specific pathogen free)-Hühnereier wurden 12 Tage bei 37°C bebrütet, mit einem Schliergerät auf erfolgtes Embryowachstum geprüft, mit Alkohol desinfiziert und in einer Sterilbox geöffnet. Die Embryonen wurden entnommen, gewaschen und mit einer Schneidevorrichtung grob zerkleinert. Anschließend erfolgte ein enzymatischer Verdau des embryonalen Gewebes durch Zugabe von proteolytischen Enzymen (1 mg/Embryo) bei 37°C während 20 Minuten. Aus dieser Gewebesuspension wurden Gewebestücke > 1000 µm über ein Sieb mit > 1000 µm entsprechender Maschenweite abgetrennt. Eine weitere Auftrennung dieser Zellaggregate konnte durch ein Sedimentationsverfahren erreicht werden, wobei als Trennkriterium eine Sedimentationsrate von ≧ 1 cm/min. gewählt wurde. Dazu wurde die Zellsuspension mit einer Flußrate von 1 cm/min. von unten nach oben durch ein Sedimentationsgefäß gepumpt. Einzelzellen bzw. kleine Zellaggregate blieben in Suspension, während größere Zellverbände sich am Boden des Gefäßes absetzten. Untersuchungen mittels Sieben verschiedener Porengrößen zeigten, daß die sich unter diesen Bedingungen absetzenden Zellverbände eine Größenverteilung von < 1000 µm, > 100 µm aufwiesen. Die Größe der in Suspension verbleibenden Zellverbände war demnach < 100 µm.

Auf diese Weise wurden drei Fraktionen von Zellaggregaten erhalten
1. < 100 µm
2. > 1000 µm
3. < 1000 µm > 100 µm
Von diesen Fraktionen wurden jeweils gleiche Biomasse-Konzentrationen (30 g pro Liter) in einem Kulturmedium (Med 199) suspendiert und mit FSME Virus (1 x 10⁵ pfu pro mg Biomasse) infiziert. Die Virusvermehrung erfolgte bei 37°C, wobei die Zellen unter gleichmäßigem Rühren in Suspension gehalten wurden. Nach vier Tagen wurde die gebildete Virusantigenmenge mittels ELISA bestimmt.

Im Falle der Fraktion 1 (Zellaggregate kleiner als 100µm) betrug die Virus/Virusantigenproduktion 4,9 µg/ml; im Falle der Fraktion 2 (Zellaggregate größer als 1000µm) betrug die Virus/Virusantigenproduktion 4,7 µg/ml; im Falle der erfindungsgemäß verwendeten Fraktion betrug die Virus/Virusantigenproduktion 9,3 µg/ml.

### Beispiel 2: Einfluß der Zellaggregatgröße auf die Verunreinigung mit CEC(chick embryo cell)Protein

Die im Beispiel 1 erhaltenen Kulturmedien der Fraktionen 1 und 3 wurden zwei Tage nach Beginn der Kultivierung auf ihren Gehalt an CEC-Protein untersucht. Das Kulturmedium der Fraktion 1 enthielt 1,80 mg CEC-Protein/ml, während im Kulturmedium der Fraktion 3 lediglich 0,84 mg CEC-Protein/ml festgestellt wurden.

### Beispiel 3: Einfluß der Menge an Saatvirus auf die Virus/Antigenproduktion

Es wurden, wie in Beispiel 1 beschrieben, Biomassen mit Zellaggregatgrößen der Fraktionen 1 und 3 kultiviert und die Virus/Antigenproduktion bestimmt, wobei pro Fraktion vier verschieden hohe Mengen an Saatvirus (3,2.10³ pfu, 1.10⁴ pfu, 3,2.10⁴ pfu und 1.10⁵ pfu, jeweils pro mg Biomasse) zur Infektion verwendet wurden. Die Ergebnisse sind aus der folgenden Tabelle 1 zu ersehen, woraus sich ergibt, daß die erfindungsgemäß verwendete Fraktion 3 bei gleicher Menge an Saatvirus eine wesentlich größere Produktion an Virus/Virusantigen aufweist.

**Tabelle 1**

| Menge an Saatvirus zur Infektion (pfu pro mg Biomasse) | Virus/Virusantigenausbeute (µg/ml) | |
|---|---|---|
| | Fraktion 1 | Fraktion 3 |
| 3,2 . 10³ pfu | 2,0 µg/ml | 8,8 µg/ml |
| 1 . 10⁴ pfu | 3,2 µg/ml | 10,3 µg/ml |
| 3,2 . 10⁴ pfu | 5,0 µg/ml | 11,0 µg/ml |
| 1 . 10⁵ pfu | 5,0 µg/ml | 11,0 µg/ml |

### Beispiel 4: Einfluß der Sauerstoffkonzentration auf die Antigenproduktion

Gemäß den in Beispiel 1 angegebenen Bedingungen wurde die erfindungsgemäße Biomasse bei verschiedenen Sauerstoffkonzentrationen im Kulturmedium kultiviert und die Menge an Virus/Virusantigen bestimmt. Die Ergebnisse sind in der nachfolgenden Tabelle 2 wiedergegeben, wobei die Ausbeute an Virus/Virusantigen unter einer Sauerstoffkonzentration von 0,06 mmol/l mit 100% gerechnet wurde.

**Tabelle 2**

| Sauerstoffkonzentration | Ausbeute an Virus/Virusantigen |
|---|---|
| 0,06 mmol/l | 100% |
| 0,02 mmol/l | 90% |
| 0,004 mmol/l | 35% |
| 0 | 10% |

### Beispiel 5: Einfluß des Sauerstofftransfers auf die Virus/Antigenausbeute

Es wurde, wie in Beispiel 1 beschrieben, Biomasse mit den Zellaggregaten der Fraktion 3 mit Virus infiziert, in verschieden großen Kulturgefäßen kultiviert und mit Luft begast, um verschiedene OTR-Werte zu erhalten. Die Suspension wurde 4 Tage bei einer Temperatur von 33°C - 37°C gehalten und die Virus/Antigenausbeute bestimmt. (Siehe Tabelle 3).

**Tabelle 3**

| Kulturgefäß | Arbeitsvolumen | OTR | Virus/Antigenausbeute |
|---|---|---|---|
| 0,5 l Spinner (Technespinner) | 0,1 | 2,38 | 5,9 |
| | 0,3 | 0,795 | 0,97 |
| 2 l Spinner (Technespinner) | 0,3 | 1,65 | 2,0 |
| | 1,0 | 0,49 | 0,32 |
| 10 l Rührflasche (Rührerlänge 5,5 cm, 150 - 160 Upm) | 3,0 | 0,41 | 0,38 |
| 50 l Rührflasche (Rührerlänge 15 cm, 80 - 90 Upm) | 18 | 1,10 | 0,97 |
| | 30 | 0,87 | 0,22 |
| Arbeitsvolumen: Liter, OTR: mmol O₂.l⁻¹.h⁻¹, Virus/Antigenausbeute: µg/ml (bestimmt mittels ELISA). | | | |

Aus den in Tabelle 3 angegebenen Ergebnissen kann abgeleitet werden, daß bei einer OTR von größer als 2 Virus/Antigenausbeuten von etwa 6 µg/ml erzielbar sind.

## Patentansprüche (Patentansprüche für folgende(n) Vertragsstaat(en): AT, BE, CH, DE, DK, FR, GB, GR, IT, LI, LU, NL, SE)

1. Biomasse zur Produktion von Virus/Virusantigen, dadurch gekennzeichnet, daß die Biomasse aus Zellaggregaten mit einem Durchmesser zwischen 100µm und 1000µm besteht, welche Biomasse mit Virus infiziert ist.

2. Biomasse nach Anspruch 1, dadurch gekennzeichnet, daß die Zellaggregate durch mechanische und enzymatische Behandlung menschlicher oder tierischer Gewebe erhalten sind.

3. Biomasse nach Anspruch 1, dadurch gekennzeichnet, daß die Zellaggregate aus menschlichen oder tierischen Einzelzellen durch Behandeln mit zellaggregierenden Substanzen erhalten sind.

4. Biomasse nach Anspruch 1, dadurch gekennzeichnet, daß die metabolische Aktivität der im Kulturmedium suspendierten Biomasse, gemessen am Glucoseverbrauch, pro Stunde zwischen 3 und 5 mg Glucose pro Gramm Biomasse beträgt.

5. Verfahren zur Produktion von Virus/Virusantigen unter Verwendung einer Biomasse nach einem oder mehreren der Ansprüche 1 bis 4, dadurch gekennzeichnet, daß es in einem Kulturmedium bei einer Sauerstoffkonzentration von mindestens 0,01 mmol/l, vorzugsweise von mindestens 0,06 mmol/l, durchgeführt wird.

6. Verfahren nach Anspruch 5, dadurch gekennzeichnet, daß es in einem Kulturmedium durchgeführt wird, welches mindestens 10mg Zellaggregate pro ml aufweist.

7. Verfahren zur Produktion von Frühsommer-Meningoenzephalitis-Virus(FSME-Virus)/Virusantigen nach Anspruch 5, dadurch gekennzeichnet, daß eine Biomasse bestehend aus Zellaggregaten von Vogelembryonalzellen, insbesondere von Hühnerembryonalzellen, als Kulturmedium verwendet wird.

8. Verfahren nach Anspruch 7, dadurch gekennzeichnet, daß im Kulturmedium eine Sauerstoff-Transfer-Rate von größer als 1,60 mmol O₂.l⁻¹.h⁻¹, vorzugsweise zwischen 1,65 und 2,40 mmol O₂.l⁻¹.h⁻¹, eingestellt wird.

9. Verfahren nach Anspruch 8, dadurch gekennzeichnet, daß bei einer Sauerstoff-Transfer-Rate von 1,65 mmol O₂.l⁻¹.h⁻¹ eine Biomasse von maximal 30 g pro Liter Kulturmedium eingestellt wird.

10. Verfahren zur Produktion von Influenza-, Vaccinia- oder Avipox-Virus/Virusantigen nach Anspruch 5, dadurch gekennzeichnet, daß eine Biomasse bestehend aus Zellagregaten von Vogelembryonalzellen als Kulturmedium verwendet wird.

11. Verwendung von Zellaggregaten mit einem Durchmesser zwischen 100µm und 1000µm zur Herstellung einer Biomasse nach Anspruch 1.

## Patentansprüche (Patentansprüche für folgende(n) Vertragsstaat(en): ES)

1. Verfahren zur Herstellung einer Biomasse zur Produktion von Virus/Virusantigen, dadurch gekennzeichnet, daß die Größe der Zellaggregate der Biomasse auf einen Durchmesser zwischen 100 µm und 1000 µm eingestellt wird und die Biomasse mit Virus infiziert wird.

2. Verfahren nach Anspruch 1, dadurch gekennzeichnet, daß die Größe der Zellaggregate durch mechanische und enzymatische Bahandlung menschlicher oder tierischer Gewebe eingestellt wird.

3. Verfahren nach Anspruch 1, dadurch gekennzeichnet, daß die Größe der Zellaggregate durch Behandeln von menschlichen oder tierischen Einzelzellen mit zellaggregierenden Substanzen eingestellt wird.

4. Verfahren nach Anspruch 1, dadurch gekennzeichnet, daß die metabolische Aktivität der im Kulturmedium suspendierten Biomasse, gemessen am Glukoseverbrauch, auf zwischen 3 und 5 mg Glucose pro Gramm Biomasse und Stunde eingestellt wird.

5. Verfahren zur Produktion von Virus/Virusantigen unter Verwendung einer Biomasse hergestellt nach einem oder mehreren der Ansprüche 1 bis 4, dadurch gekennzeichnet, daß es in einem Kulturmedium bei einer Sauerstoffkonzentration von mindestens 0,01 mmol/l, vorzugsweise von mindestens 0,06 mmol/l, durchgeführt wird.

6. Verfahren nach Anspruch 5, dadurch gekennzeichnet, daß es in einem Kulturmedium durchgeführt wird, welches mindestens 10 mg Zellaggregate pro ml aufweist.

7. Verfahren zur Produktion von Frühsommer-Meningoenzephalitis-Virus(FSME-Virus)/Virusantigen nach Anspruch 5, dadurch gekennzeichnet, daß eine Biomasse bestehend aus Zellaggregaten von Vogelembryonalzellen, inbesondere von Hühnerembryonalzellen, als Kulturmedium verwendet wird.

8. Verfahren nach Anspruch 7, dadurch gekennzeichnet, daß im Kulturmedium eine Sauerstoff-Transfer-Rate von größer als 1,60 mmol O₂.l⁻¹.h⁻¹, vorzugsweise zwischen 1,65 und 2,40 mmol O₂.l⁻¹.h⁻¹, eingestellt wird.

9. Verfahren nach Anspruch 8, dadurch gekennzeichnet, daß bei einer Sauerstoff-Transfer-Rate von 1,65 mmol O₂.l⁻¹.h⁻¹ eine Biomasse von maximal 30 g pro Liter Kulturmedium eingestellt wird.

10. Verfahren zur Produktion von Influenza-, Vaccinia- oder Avipox-Virus/Virusantigen nach Anspruch 5, dadurch gekennzeichnet, daß eine Biomasse bestehend aus Zellaggregaten von Vogelembryonalzellen als Kulturmedium verwendet wird.

11. Verwendung von Zellaggregaten mit einem Druchmesser zwischen 100 µm und 1000 µm zur Herstellung einer Biomasse nach Anspruch 1.

## Claims (Claims for the following Contracting State(s): AT, BE, CH, DE, DK, FR, GB, GR, IT, LI, LU, NL, SE)

1. A biomass for producing virus/virus antigen, characterized in that the biomass is comprised of cell aggregates having diameters of between 100 µm and 1,000 µm, which biomass is infected with virus.

2. A biomass according to claim 1, characterized in that the cell aggregates are obtained by mechanic and enzymatic treatment of human or animal tissues.

3. A biomass according to claim 1, characterized in that the cell aggregates are obtained from human or animal single cells by treatment with cell aggregating substances.

4. A biomass according to claim 1, characterized in that the metabolic activity of the biomass suspended in culture medium, based on the glucose consumption, is between 3 and 5 mg of glucose per gram of biomass per hour.

5. A method of producing virus/virus antigen by using the biomass according to one or several of claims 1 to 4, characterized in that it is carried out in a culture medium at an oxygen concentration of at least 0.01 mmol/l, preferably of at least 0.06 mmol/l.

6. A method according to claim 5, characterized in that it is carried out in a culture medium containing at least 10 mg cell aggregates per ml.

7. A method for producing tick-borne encephalitis virus (TBE)-virus/virus antigen according to claim 5, characterized in that a biomass comprising cell aggregates of avian embryo cells, in particular chick embryo cells, is used as said culture medium.

8. A method according to claim 7, characterized in that an oxygen transfer rate of more than 1.60 mmol O₂.l⁻¹.h⁻¹, preferably of between 1.65 and 2.40 mmol O₂.l⁻¹.h⁻¹, is adjusted in the culture medium.

9. A method according to claim 8, characterized in that, at an oxygen transfer rate of 1.65 mmol O₂.l⁻¹.h⁻¹, a biomass of maximally 30 g per liter of culture medium is adjusted.

10. A method of producing influenca, vaccinia or avipox virus/virus antigen according to claim 5, characterized in that a biomass comprised of cell aggregates of avian embryo cells is used as said culture medium.

11. The use of cell aggregates having diameters of between 100 µm and 1,000 µm for producing a biomass according to claim 1.

## Claims (Claims for the following Contracting State(s): ES)

1. A method for obtaining a biomass for the production of virus/virus antigen, characterized in that the size of the cell aggregates of the biomass is adjusted to a diameter of between 100 µm and 1,000 µm and the biomass is infected with virus.

2. A method according to claim 1, characterized in that the size of the cell aggregates is adjusted by mechanic and enzymatic treatment of human or animal tissues.

3. A method according to claim 1, characterized in that the size of the cell aggregates is adjusted by treatment of human or animal single cells with cell aggregating substances.

4. A method according to claim 1, characterized in that the metabolic activity of the biomass suspended in culture medium, based on the glucose consumption, is adjusted to between 3 and 5 mg of glucose per gram of biomass per hour.

5. A method of producing virus/virus antigen by using the biomass according to one or several of claims 1 to 4, characterized in that it is carried out in a culture medium at an oxygen concentration of at least 0.01 mmol/l, preferably of at least 0.06 mmol/l.

6. A method according to claim 5, characterized in that it is carried out in a culture medium containing at least 10 mg cell aggregates per ml.

7. A method for producing tick-borne encephalitis virus (TBE)-virus/virus antigen according to claim 5, characterized in that a biomass comprising cell aggregates of avian embryo cells, in particular chick embryo cells, is used as said culture medium.

8. A method according to claim 7, characterized in that an oxygen transfer rate of more than 1.60 mmol O₂.l⁻¹.h⁻¹, preferably of between 1.65 and 2.40 mmol O₂.l⁻¹.h⁻¹, is adjusted in the culture medium.

9. A method according to claim 8, characterized in that, at an oxygen transfer rate of 1.65 mmol O₂.l⁻¹.h⁻¹, a biomass of maximally 30 g per liter of culture medium is adjusted.

10. A method of producing influenca, vaccinia or avipox virus/virus antigen according to claim 5, characterized in that a biomass comprised of cell aggregates of avian embryo cells is used as said culture medium.

11. The use of cell aggregates having diameters of between 100 µm and 1,000 µm for producing a biomass according to claim 1.

## Revendications (Revendications pour l'(les) Etat(s) contractant(s) suivant(s): AT, BE, CH, DE, DK, FR, GB, GR, IT, LI, LU, NL, SE)

1. Biomasse pour la fabrication de virus/antigène de virus, caractérisée en ce que ladite biomasse est constituée d'agrégats de cellules ayant un diamètre compris entre 100 µm et 1000 µm, laquelle biomasse est infectée de virus.

2. Biomasse selon la revendication 1, caractérisée en ce que les agrégats de cellules sont obtenus par traitement mécanique et enzymatique de tissus humains ou animaux.

3. Biomasse selon la revendication 1, caractérisée en ce que les agrégats de cellules sont obtenus à partir de cellules humaines ou animales individuelles par traitement avec des substances agrégant des cellules.

4. Biomasse selon la revendication 1, caractérisée en ce que l'activité métabolique de la biomasse suspendue dans le milieu de culture, mesurée sur la consommation de la glucose, est comprise entre 3 et 5 mg de glucose par gramme de biomasse par heure.

5. Procédé pour la fabrication de virus/antigène de virus en utilisant une biomasse selon l'une ou plusieurs des revendications 1 à 4, caractérisé en ce qu'il est effectué au sein d'un milieu de culture à une concentration d'oxygène d'au moins 0,01 mmole/l, de préference d'au moins 0,06 mmole/l.

6. Procédé selon la revendication 5, caractérisé en ce qu'il est effectué au sein d'un milieu de culture comprenant au moins 10 mg d'agrégats de cellules par ml.

7. Procédé pour la fabrication de virus de la méningoencéphalite du début de l'été (virus FSME)/antigène de virus selon la revendication 5, caractérisé en ce qu'une biomasse constituée d'agrégats de cellules d'embryons d'oiseaux, notamment de cellules d'embryons de poulet, est utilisée en tant que milieu de culture.

8. Procédé selon la revendication 7, caractérisé en ce qu'un débit de transfert d'oxygène supérieure à 1,60 mmole O₂.l⁻¹.h⁻¹, de préference compris entre 1,65 et 2,40 mmole O₂.l⁻¹.h⁻¹, est ajusté.

9. Procédé selon la revendication 8, caractérisé en ce que, à un débit de transfert d'oxygène de 1,65 mmole O₂.l⁻¹.h⁻¹, une biomasse de 30 g par litre de milieu de culture au maximum est ajustée.

10. Procédé pour la fabrication de virus d'influenza, de vaccinia ou d'avipox/antigène de virus selon la revendication 5, caractérisé en ce qu'une biomasse consistant en d'agrégats de cellules d'embryons d'oiseaux est utilisée en tant que milieu de culture.

11. Utilisation d'agrégats de cellules ayant un diamètre compris entre 100 µm et 1000 µm pour la fabrication d'une biomasse selon la revendication 1.

## Revendications (Revendications pour l'(les) Etat(s) contractant(s) suivant(s): ES)

1. Procédé pour l'obtention d'une biomasse pour la fabrication de virus/antigène de virus, caractérisé en ce que la taille des agrégats de cellules de ladite biomasse est adjustée à un diamètre compris entre 100 µm et 1000 µm et ladite biomasse est infectée de virus.

2. Procédé selon la revendication 1, caractérisé en ce que la taille des agrégats de cellules est ajustée par traitement mécanique et enzymatique de tissus humains ou animaux.

3. Procédé selon la revendication 1, caractérisé en ce que la taille des agrégats de cellules est ajusté par traitement des cellules humaines ou animales individuelles avec des substances agrégant des cellules.

4. Procédé selon la revendication 1, caractérisé en ce que l'activité métabolique de la biomasse suspendue dans le milieu de culture, mesurée sur la consommation de la glucose, est ajustée entre 3 et 5 mg de glucose par gramme de biomasse par heure.

5. Procédé pour la fabrication de virus/antigène de virus en utilisant une biomasse fabriquée selon l'une ou plusieurs des revendications 1 à 4, caractérisé en ce qu'il est effectué au sein d'un milieu de culture à une concentration d'oxygène d'au moins 0,01 mmole/l, de préference d'au moins 0,06 mmole/l.

6. Procédé selon la revendication 5, caractérisé en ce qu'il est effectué au sein d'un milieu de culture comprenant au moins 10 mg d'agrégats de cellules par ml.

7. Procédé pour la fabrication de virus de la méningoencéphalite du début de l'été (virus FSME)/antigène de virus selon la revendication 5, caractérisé en ce qu'une biomasse constituée d'agrégats de cellules d'embryons d'oiseaux, notamment de cellules d'embryons de poulet, est utilisée en tant que milieu de culture.

8. Procédé selon la revendication 7, caractérisé en ce qu'un débit de transfert d'oxygène supérieure à 1,60 mmole O₂.l⁻¹.h⁻¹, de préference compris entre 1,65 et 2,40 mmole O₂.l⁻¹.h⁻¹, est ajusté.

9. Procédé selon la revendication 8, caractérisé en ce que, à un débit de transfert d'oxygène de 1,65 mmole O₂.l⁻¹.h⁻¹, une biomasse de 30 g par litre de milieu de culture au maximum est ajustée.

10. Procédé pour la fabrication de virus d'influenza, de vaccinia ou d'avipox/antigène de virus selon la revendication 5, caractérisé en ce qu'une biomasse consistant en d'agrégats de cellules d'embryons d'oiseaux est utilisée en tant que milieu de culture.

11. Utilisation d'agrégats de cellules ayant un diamètre compris entre 100 µm et 1000 µm pour la fabrication d'une biomasse selon la revendication 1.
